# EUROPEAN PATENT SPECIFICATION

(11) **EP 3 119 400 B1**
(45) Date of publication and mention of the grant of the patent: **02.06.2021**
(21) Application number: 15710216.1
(22) Date of filing: 18.03.2015
(51) Int. Cl.: A61K 31/525, A61P 3/00, A61P 3/04, A61P 3/08, A61P 3/10, A23L 33/135, A23L 33/15, A23L 33/16

(54) **MATERNAL VITAMIN B2 ADMINISTRATION FOR THE PREVENTION OF INCREASED ADIPOSITY, OVERWEIGHT OR OBESITY IN THE OFFSPRING**
MATERNALE VERABREICHUNG VON VITAMIN B2 ZUR VERHINDERUNG VON ERHÖHTER ADIPOSITAS, ÜBERGEWICHT ODER FETTLEIBIGKEIT BEIM NACHWUCHS
ADMINISTRATION MATERNELLE DE VITAMINE B2 POUR LA PRÉVENTION DE L'ADIPOSITÉ ACCRUE, DU SURPOIDS OU DE L'OBÉSITÉ CHEZ L'ENFANT

(30) Priority: 21.03.2014 EP 14161190
(43) Date of publication of application: 25.01.2017
(73) Proprietor: Société des Produits Nestlé S.A., 1800 Vevey (CH)
(72) Inventor: COOPER, Cyrus, Southampton Hampshire SO16 7HW (GB); GLUCKMAN, Peter David, Auckland 1050 (NZ); GODFREY, Keith Malcolm, Hampshire SO40 7AP (GB); MACE, Catherine, CH-1005 Lausanne (CH); SILVA ZOLEZZI, Irma, CH-1804 Carrouge (CH)
(74) Representative: Gagliardi, Tatiana
(86) International application number: PCT/EP2015/055732
(87) International publication number: WO 2015/140233

(56) References cited:
- EP-A1- 2 147 678
- WO-A1-2007/135141
- WO-A1-2014/016627
- ERIC D CIAPPIO ET AL: "Maternal one-carbon nutrient intake and cancer risk in offspring", NUTRITION REVIEWS, vol. 69, no. 10, 1 October 2011 (2011-10-01), pages 561-571, XP055117815, ISSN: 0029-6643, DOI: 10.1111/j.1753-4887.2011.00424.x
- YAJNIK C S ET AL: "Vitamin B12 and folate concentrations during pregnancy and insulin resistance in the offspring: the Pune Maternal Nutrition Study", DIABETOLOGIA ; CLINICAL AND EXPERIMENTAL DIABETES AND METABOLISM, SPRINGER, BERLIN, DE, vol. 51, no. 1, 13 September 2007 (2007-09-13), pages 29-38, XP019558636, ISSN: 1432-0428, DOI: 10.1007/S00125-007-0793-Y
- S. R. CROZIER ET AL: "Maternal vitamin D status in pregnancy is associated with adiposity in the offspring: findings from the Southampton Women's Survey", AMERICAN JOURNAL OF CLINICAL NUTRITION, vol. 96, no. 1, 23 May 2012 (2012-05-23), pages 57-63, XP055117634, ISSN: 0002-9165, DOI: 10.3945/ajcn.112.037473

## Description

The present invention generally relates to the early prevention of overweight, obesity, excessive fat accumulation and associated metabolic disorders in infants or children. For example, the present invention generally relates to the prevention of overweight, obesity excessive fat accumulation and/or associated metabolic disorders in infants or children through appropriate maternal nutrition before pregnancy, during pregnancy and/or during lactation. Embodiments of the present invention relate to vitamin B2 for use in the prevention of overweight, obesity, excessive fat accumulation and/or associated metabolic disorders in the offspring, as defined in the appended claims, wherein the vitamin B2 is administered to the mother before pregnancy, during pregnancy and/or during lactation and to maternal food compositions that can be used for this purpose.

### Background

Scientific evidence has accumulated showing that prenatal and post natal early nutrition and other environmental factors cause programming of long-term health and well-being, and can impact the risk of developing chronic diseases. Several studies have shown that changes in dietary intake or manipulation of individual macro and micronutrients during the reproductive period can have an impact in several physiological processes, such as growth, metabolism, appetite, cardiovascular function among others (Koletzko B et al (2011) Am J Nutr 94(s):2036-43S). Therefore nutritional status (nutrient stores and dietary intake) of women before and during pregnancy is of relevance to optimize neonatal and child health outcomes. Maternal nutrition is thought to affect the availability and supply of nutrients to the developing fetus that are required for critical developmental processes.

Childhood overweight and obesity are major public health problem in a wide range of countries (including middle and low-income countries) and increasing rates of overweight and obesity have been reported in the last three decades. In 2008 in the UK about 30% of children 2-15 years old were overweight or obese. Evidence shows that weight at 5 years of age is good indicator of future health and well-being of a child (Gardner et al (2009) Pediatrics 123:e67-73). It has been shown that obesity in childhood increases the risk of adult obesity and other highly detrimental chronic conditions such as cardiovascular disease, type 2 diabetes, hepatic, renal and musculoskeletal complications, etc, among others. There is strong evidence that once obesity is established it is difficult to reverse through interventions and continues till adulthood (Waters E et al. (2011) Cochrane Database of Systematic Reviews 12), underlining the importance of childhood obesity prevention efforts.

Some possible early-life determinants including maternal obesity and diabetes, excess gestational weight gain, maternal smoking, rapid infant growth have been clearly associated with later in life overweight and obesity (Monasta L et al. (2010) Obesity Reviews. 11:695-708). Although the association may be modest for each of these factors, a large effect may be achieved when acting on a small attributable risk if the risk factor is highly prevalent in a population. Also some possible determinants may become more important than others because they are easier to be addressed through the implementation of an effective intervention.

Micronutrient deficiencies have profound and often persistent effects on fetal tissues and organs, even in the absence of clinical signs of their deficiency in the mother (Ashworth CJ et al (2001) 122:527-35). Inadequate intakes of multiple micronutrients are common among women of reproductive age living in resource poor-settings (Torhem LE et al. (2010) J. Nutr. 140: 2051S-58S), and in some settings malnourishment related to overweight and obesity are also emerging concerns due to poor diet.

WO 2007/135141 describes the use of docosahexaenoic acid in the manufacture of a composition for administration to a pregnant woman for reducing the risk of development of overweight or obesity of the baby in infancy and/or early childhood. The inventors have investigated micronutrient deficiencies in women in order to identify micronutrients that can be used in prenatal and post natal early nutrition to program long-term health and well-being, and that - in particular - have a positive impact on reducing the risk of developing chronic diseases, such as overweight, obesity, excessive fat accumulation and associated metabolic disorders such as diabetes, cardiovascular diseases and hypertension.

Any reference to prior art documents in this specification is not to be considered an admission that such prior art is widely known or forms part of the common general knowledge in the field.

It would therefore be desirable to provide the art with a solution that allows it to reduce the likelihood of developing overweight, obesity, excessive fat accumulation and/or associated disorders as early in life as possible.

One object of the present invention is to improve the state of the art and in particular to provide a solution that overcomes at least some of the disadvantages of the present state of the art and that satisfies the needs expressed above, or to at least provide a useful alternative.

### Summary of the invention :

The invention relates to Vitamin B2 for use in the therapeutic prevention of overweight, obesity, excessive fat accumulation and metabolic disorders associated with either of the foregoing in the offspring, wherein the vitamin B2 is to be administered to the mother before pregnancy, during pregnancy and/or during lactation wherein associated metabolic disorders in the offspring are selected from the group consisting of: cardiovascular diseases such as coronary heart disease; insulin resistance; type 2 diabetes; hypertension; sleep apnea, respiratory problems and /or dyslipidemia; stroke; liver and gallbladder disease; osteoarthritis; and/or gynecological problems.

### Description of the invention

The invention is defined by the claims. Any subject-matter falling outside the scope of the claims is provided for information purposes only. The present inventors were surprised to see that they could achieve the above objectives by the subject matter of the independent claims. The dependent claims further develop the idea of the present invention.

The present inventors have investigated the role of epigenetics as a mediator and a marker of early nutritional effects on human childhood body composition and the risk of humans developing obesity and insulin-resistance related disorders later in life. They have conducted thorough and detailed analyses in multi-cohort studies and were surprised to find that a vitamin B2 deficiency in expecting mothers was significantly correlated with an increased likelihood that the offspring develops overweight, obesity, excessive fat accumulation and metabolic disorders associated with the foregoing.

Consequently, the present invention relates in part to vitamin B2 for use in the therapeutic prevention of overweight, obesity, excessive fat accumulation and metabolic disorders associated with either of the foregoing in the offspring, wherein the vitamin B2 is to be administered to the mother before pregnancy, during pregnancy and/or during lactation wherein associated metabolic disorders in the offspring are selected from the group consisting of: cardiovascular diseases such as coronary heart disease; insulin resistance; type 2 diabetes; hypertension; sleep apnea, respiratory problems and /or dyslipidemia; stroke; liver and gallbladder disease; osteoarthritis; and/or gynecological problems.

The present disclosure also relates to the use of vitamin B2 for the preparation of a composition for the prevention of overweight, obesity, excessive fat accumulation and metabolic disorders associated with either of the foregoing in the offspring.

The present disclosure further relates to vitamin B2 for use in the reduction of the likelihood for the development of overweight, obesity, excessive fat accumulation and metabolic disorders associated with either of the foregoing in the offspring.

Alternatively, according to any embodiment described herein, vitamin B2 or a composition comprising vitamin B2 is used to prevent or to reduce the likelihood for the development of overweight, obesity, associated metabolic disorders and/or excessive fat accumulation.

The vitamin B2 is to be administered to the mother before pregnancy, during pregnancy and/or during lactation.

Vitamin B2 supplementation is in particular effective in mothers which have low levels of vitamin B2 and/or have insufficient vitamin B2 intake.

Vitamin B2 intake is considered insufficient if it is below the Recommended Dietary Allowance (RDA). The RDA is the daily dietary intake level of a nutrient considered sufficient to meet the requirements of 97.5% of healthy individuals in each life-stage and gender group. It is calculated based on the Estimated Average Requirements (EAR), which are expected to satisfy the needs of 50% of the people in that age group based on a review of the scientific literature.

For example, the Vitamin B2 intake of a pregnant woman may be considered insufficient if it is below 1.4 mg/day.

Presently, vitamin B2 supplementation is used in the art, e.g., for preventing migraine headaches, acne, muscle cramps, or for eye conditions such as eye fatigue. "Overweight" is defined for an adult human as having a BMI between 25 and 30.

"Body mass index" or "BMI" means the ratio of weight in kg divided by the height in meters, squared.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

For children the BMI is plotted on a BMI vs. age growth chart (for either girls or boys) to obtain a percentile ranking. Percentiles are the most commonly used indicator to assess the size and growth patterns of individual children. The percentile indicates the relative position of the child's BMI among children of the same sex and age. Children are considered overweight if their BMI is located between the 85^{th} and 95^{th} percentile. Children are considered obese if their BMI is located on or above the 95^{th} percentile.

Metabolic disorders that are associated with overweight, obesity and/or excessive fat accumulation are similar and well known to skilled artisans. For example, these disorders include cardiovascular diseases such as coronary heart disease; insulin resistance; type 2 diabetes; hypertension; sleep apnea, respiratory problems and /or dyslipidemia; but also some cancers such as endometrial, breast, and/or colon cancer; stroke; liver and gallbladder disease; osteoarthritis; and/or gynecological problems.

As vitamin B2 may be administered to expecting mothers during pregnancy and/or to mothers during lactation it may for example be to be administered in the form of a maternal food composition.

Women's nutrient needs increase during pregnancy and lactation. If the increased nutrient needs are satisfied this protects maternal and infant health. Lactation is demanding on maternal stores of energy, protein, and other nutrients that need to be established, and replenished.

Maternal food compositions are food compositions designed to help meeting the specific nutritional requirements of women during pregnancy and lactation.

For example, such maternal food compositions may comprise sources of protein, iron, iodine, vitamin A, and/or folate.

The maternal food composition may have any form that is accepted by mothers as part of their diet or as nutritional supplement.

For example, the maternal food composition may be selected from the group consisting of a powdered nutritional composition to be reconstituted in milk or water, a nutritional formula, a cereal based-product, a drink, a bar, a nutritional supplement, a nutraceutical, a yogurt, a milk-derived product, a food sprinkler, a pill or a tablet.

Currently, particularly well accepted by consumers are powdered nutritional compositions to be reconstituted in milk or water.

Also well accepted are nutritional supplements, for example in the form of a tablet. The supplement provides selected nutrients while not representing a significant portion of the overall nutritional needs of the subject and/or does not represent more than 0.1%, 1%, 5%, 10%, or 20% of the daily energy need of the subject

Vitamin B2 may be used in any amount that is effective in achieving the objective of the present invention. Skilled artisans will be able to determine appropriate dosages. Typically, dosage will depend on age, size and health status of the mother, on her lifestyle as well as on her genetic heritage.

In the prophylactic applications of the present invention, Vitamin B2 is administered in an amount that is sufficient to at least partially reduce the risk of the development of overweight, obesity, excessive fat accumulation and/or associated metabolic disorders in the offspring. Such an amount is defined to be "a prophylactic effective dose". Hence, vitamin B2 may be administered in a prophylactic effective dose.

For example, the vitamin B2 is administered in an amount corresponding to 0.14-14 mg Vitamin B2/day, for example in an amount corresponding to 0.5 - 2 mg Vitamin B2/day.

For the purpose of the present invention it is preferred if Vitamin B2 is administered regularly, for example two times a day, daily, every two days, or weekly.

The vitamin B2 may be provided as a sustained release formulation. This way, vitamin B2 can be consumed less frequently, while the body is still constantly supplied with sufficient Vitamin B2.

For example the vitamin B2 may be to be administered before pregnancy (pre-pregnancy), during part of or the whole pregnancy and/or during the breastfeeding period (lactation). In one embodiment vitamin B2 may be administered during pregnancy and/or during lactation.

In one embodiment the composition of the invention is administered before pregnancy, for example during the 1, 2, or 4 months preceding the pregnancy or desired pregnancy.

As the nutritional requirements increase in the second and particularly the third trimester of pregnancy, it may be preferred to administer Vitamin B2 regularly throughout the third trimester of pregnancy or throughout the second and third trimester of pregnancy.

For example, vitamin B2 may be to be administered daily. The regular administration of vitamin B2 may be continued for at least 4, at least 8, at least 12, at least 16, at least 20, at least 24, at least 28, at least 32 or at least 36 consecutive weeks during pregnancy and/or during lactation.

Vitamin B2 may be used in pure form or as a natural vitamin B2 source or an extract thereof.

Highly purified or synthetic vitamin B2 may be used. It is preferred, if vitamin B2 is provided from natural sources or as a natural source.

For example, vitamin B2 may be provided from natural sources such as milk, meat, poultry, eggs, nuts, vegetables, mushrooms, yeasts, nuts and peanuts; or extracts and/or combinations thereof.

Vitamin B2 may be used as single active ingredient.

It may also be co-administered with one or more other compounds that are active in reducing the risk of developing overweight, obesity, excessive fat accumulation and/or associated metabolic disorders in the offspring if administered to the mother before pregnancy, during pregnancy and/or during lactation.

In accordance with the present invention, vitamin B2 may be used to prevent the generation of overweight, obesity, excessive fat accumulation and/or associated metabolic disorders in the offspring later in life.

"Later in life" includes childhood and adulthood. For example, "later in life" may refer to childhood, such as to an age of at least 3 years, for example at least 4 years or at least 6 years.

The inventors have found that the subject matter of the present invention allows it in particular to prevent overweight and/or obesity by reducing and/or avoiding the excessive build-up of fat mass in the offspring, for example abdominal and/or visceral fat mass.

This is advantageous as abdominal fat is particularly strongly correlated with cardiovascular diseases as well as other metabolic and vascular diseases, such as type 2 diabetes. Visceral fat, also known as intra-abdominal fat, is located inside the peritoneal cavity, between internal organs and torso and is also strongly correlated with type 2 diabetes.

It may further be preferred to administer vitamin B2 in accordance with the present invention to mothers and/or expecting mothers whose children are particularly at risk of developing of overweight, obesity, excessive fat accumulation and/or associated metabolic disorders.

The studies leading to the present invention have shown that these may be for example multiparous, overweight and/or obese mothers and/or mothers suffering from metabolic syndrome. Parity is a term that refers to the number of times a female has given birth to a baby. A woman who has given birth two or more times is multiparous.

The present invention also relates to a maternal food composition for use as defined in the appended claims.

Consequently, the present invention relates to a maternal food composition, wherein the maternal food composition is a powdered nutritional formula comprising a protein source, a carbohydrate source, a lipid source, lecithin such as soya lecithin, a bulking agent and 0.1-9.8 mg vitamin B2/100 g dry weight for use as defined in the appended claims.

The protein source may be dried milk or dried skimmed milk. As carbohydrate source sucrose and/or maltodextrin may be used. The lipid source may be vegetable oil. Vitamins and minerals may also be added. For example, vitamins and minerals may be added in accordance with the recommendations of Government bodies such as the USRDA. For example, the composition may contain per daily dose one or more of the following micronutrients in the ranges given: 100 to 2500mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalents (RAE), 8.5 to 850mg Vitamin C, 0.14 to 14 mg Vitamin B1, 0.19-60 mg vitamin B6, 1.8 to 35 mg niacin, 0.26 to 26 µg Vitamin B12, 60 to 1000 µg folic acid, 3 to 300 µg biotin, 1.5 to 100 µg Vitamin D, 1.9 to 109 µg Vitamin E.

The formulation may also alternatively or additionally contain glucose syrup, milk fat, fish oil, magnesium citrate, choline salts and esters, probiotic cultures, prebiotic fibers, and/or ascorbyl palmitate.

Flavor compounds, such as cocoa powder or honey, for example, may be added to provide taste variations.

The composition may further contain probiotic bacteria, folic acid, calcium, iron, ARA, EPA, and/or DHA.

Further advantages and features of the present invention are apparent from the figures and non-limiting examples.

### Brief description of the figures and drawings:

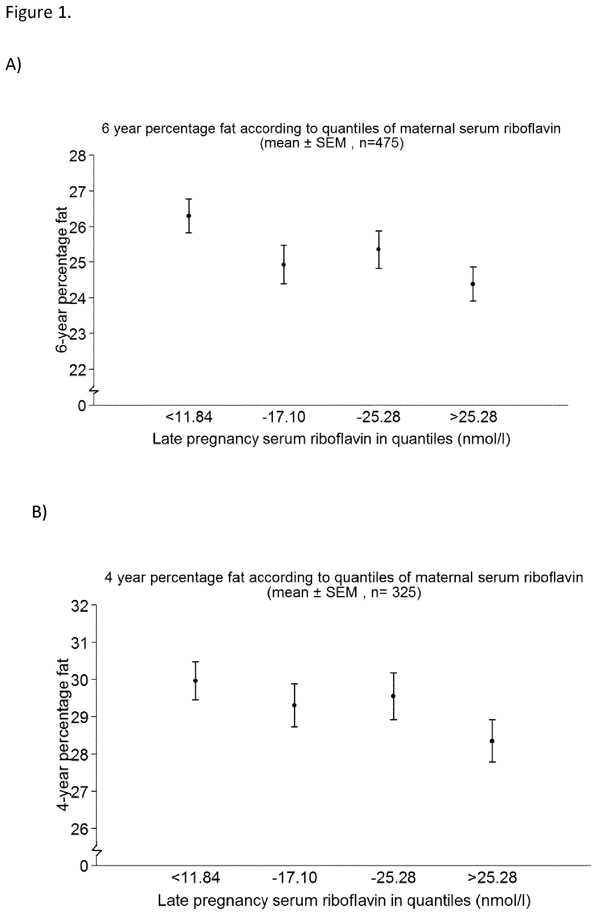

**Figure 1** shows that vitamin B2 levels in women during late pregnancy had a negative correlation with a greater adiposity of their children measured by dual X-ray absorptiometry (DXA). In panel a) higher % of fat mass values (grams) in the offspring at 6 years were significantly correlated to lower maternal vitamin B2 levels in serum and in b) a trend was observed at age 4 years.

### Examples:

Experimental and clinical research suggests that maternal nutritional state during pregnancy has lifelong effects in later in life outcomes in the offspring. In our work we seek to identify clinically and nutritionally defined groups whose offspring are at increased risk of later suboptimal body composition.

### Study design:

Mother-infant cohort included in the analysis:
501 Southampton Women's Survey (SWS) mother-infant pairs were selected as those with a late pregnancy maternal serum aliquot together with DXA measurements of body composition of the offspring at age 4 and 6 years. Summary characteristics of the SWS subjects analyzed were the following:

| | **Number** | **Percentage** |
|---|---|---|
| **Parity** | | |
| Primiparous | 244 | 48.7% |
| Multiparous | 257 | 51.3% |

| **Maternal Age (years)** | | |
|---|---|---|
| <25 | 34 | 6.8% |
| 25-35 | 398 | 79.4% |
| ≥35 | 69 | 13.8% |

| **Ethnicity** | | |
|---|---|---|
| White Caucasian | 486 | 97.0% |
| Non-white Caucasian | 15 | 3.0% |

| **Maternal Pre-pregnant BMI** | | |
|---|---|---|
| <20 kg/m2 | 32 | 6.5% |
| ≥20 kg/m2 | 464 | 93.5% |

### 1) Measurements of Vitamin B2 in maternal serum samples.

Plasma (60 µL) was deproteinized by mixing with an equal volume of 50 g/L trichloroacetic acid that contained d8-riboflavin as internal standards. Vitamin B2, was separated on a C8 reversed-phase column, which was developed with an acetonitrile gradient in a buffer containing acetic acid and heptafluorobutyric acid. The analytes were detected by tandem mass spectrometry in the positive-ion mode. [Midttun 0 et al. Multianalyte Quantification of Vitamin B6 and B2 Species in the Nanomolar Range in Human Plasma by Liquid Chromatography-Tandem Mass Spectrometry. Clinical Chemistry2005; 61(7):1206-1216.]

### 2) Statistical analyses to uncover the correlation of the vitamin B2 levels to childhood adiposity in SWS.

In the SWS children's percentage fat mass at age 6 years (but not at age 4 years) was positively skewed and values transformed with the use of Fisher-Yates normal scores to a normally distributed variable with a mean of 0 and an SD of 1 [Armitage P, Berry G. Statistical methods in medical research. Oxford, United Kingdom: Blackwell Science Ltd, 2002.]. Linear regression models were fitted with body-composition variables as the outcomes and with maternal micronutrient status as the predictor, taking account of potential confounding influences. Owing to sex differences in the children's body composition, all analyses were adjusted for the sex of the child, together with the child's age. Statistical analysis was performed with the use of Stata 11.1 [StataCorp. Stata: release 11. Statistical software. College Station, TX: StataCorp LP, 2009.]

### Results:

Maternal vitamin B2 levels in late pregnancy were negatively correlated with offspring fat mass measured by DXA at 6 years and show a trend at 4 years. Results are presented as correlation coefficients.

**Table 1. Child's adiposity by maternal vitamin B2 status.**

| **Age at DXA measurement** | **Fat content percentage Vs. Vitamin B2 levels (Correlation coefficient)** | **P-value** |
|---|---|---|
| **4 yrs old (n=325)** | -0.107 | 0.054 |
| **6 yrs old (n=475)** | -0.106 | **0.021** |

The results shown in the table 1 correspond to the log-transformed values used for the statistical analysis of the measures plotted in Figure 1. Fat content percentage at 4 years distribution was not skewed so it was not log-transformed.

These results support that lower vitamin B2 status has lasting effects on the offspring's risk of obesity and provide strong support for intervening before pregnancy, during pregnancy and during lactation to improve maternal vitamin B2 status.

## Claims

1. Vitamin B2 for use in the therapeutic prevention of overweight, obesity, excessive fat accumulation and metabolic disorders associated with either of the foregoing in the offspring, wherein the vitamin B2 is to be administered to the mother before pregnancy, during pregnancy and/or during lactation wherein associated metabolic disorders in the offspring are selected from the group consisting of: cardiovascular diseases such as coronary heart disease; insulin resistance; type 2 diabetes; hypertension; sleep apnea, respiratory problems and /or dyslipidemia; stroke; liver and gallbladder disease; osteoarthritis; and/or gynecological problems.

2. Vitamin B2 for use in accordance with claim 1, wherein the vitamin B2 is administered in the form of a maternal food composition.

3. Vitamin B2 for use in accordance with claim 2, wherein the maternal food composition is selected from the group consisting of a powdered nutritional composition to be reconstituted in milk or water, a nutritional formula, a cereal based-product, a drink, a bar, a nutritional supplement, a nutraceutical, a yogurt a milk-derived product, a food sprinkler, a pill or a tablet.

4. Vitamin B2 for use in accordance with one of the preceding claims, wherein the vitamin B2 is administered in an amount corresponding to 0.14-14mg Vitamin B2/day, for example 1 - 12.0 mg Vitamin B2/day.

5. Vitamin for use in accordance with one of the preceding claims, wherein the vitamin B2 is to be administered for at least 4 consecutive weeks before pregnancy, during pregnancy and/or during lactation.

6. Vitamin B2 for use in accordance with one of the preceding claims, wherein the vitamin B2 is to be administered at least once daily.

7. Vitamin B2 for use in accordance with one of the preceding claims, wherein the vitamin B2 is provided from natural sources such as milk, meat, poultry, eggs, nuts, vegetables, mushrooms, yeasts, nuts and peanuts; or extracts and/or combinations thereof.

8. Vitamin B2 for use in accordance with one of the preceding claims, wherein overweight obesity, excessive fat accumulation and associated metabolic disorders is prevented in the offspring later in life.

9. Vitamin B2 for use in accordance with claim 8, wherein overweight and/or obesity is prevented in the offspring at the age of at least 3 years, for example at least 4 years or at least 6 years.

10. Vitamin B2 for use in accordance with one of the preceding claims, wherein overweight and/or obesity is prevented by reducing fat mass, in particular abdominal and/or visceral fat mass.

11. Vitamin B2 for use in accordance with one of the preceding claims, wherein the mother is a multiparous, overweight and/or obese mother and/or a mother suffering from metabolic syndrome.

12. Maternal food composition for use in the therapeutic prevention of overweight, obesity, excessive fat accumulation and metabolic disorders associated with either of the foregoing in the offspring, wherein the maternal food composition is to be administered to the mother before pregnancy, during pregnancy and/or during lactation wherein associated metabolic disorders in the offspring are selected from the group consisting of: cardiovascular diseases such as coronary heart disease; insulin resistance; type 2 diabetes; hypertension; sleep apnea, respiratory problems and /or dyslipidemia; stroke; liver and gallbladder disease; osteoarthritis; and/or gynecological problems, wherein the maternal food composition is a powdered nutritional formula comprising a protein source, a carbohydrate source, a lipid source, lecithin optionally from soya, bulking agents and 0.1-9.8 mg vitamin B2/100 g dry weight, and wherein vitamin B2 is comprised in the maternal food composition as active ingredient.

13. Maternal food composition for use in in accordance with claim 12, further comprising probiotic bacteria, folic acid, calcium, iron, ARA, EPA and/or DHA.

## Patentansprüche

1. Vitamin B2 zur Verwendung bei der therapeutischen Vorbeugung von Übergewicht, Fettleibigkeit, übermäßiger Fettansammlung und Stoffwechselstörungen, die mit einem der Vorstehenden verbunden sind, bei den Nachkommen, wobei das Vitamin B2 der Mutter vor Schwangerschaft, während Schwangerschaft und/oder während Stillzeit zu verabreichen ist, wobei damit verbundene Stoffwechselstörungen bei den Nachkommen ausgewählt sind aus der Gruppe bestehend aus: kardiovaskulären Erkrankungen, wie koronarer Herzerkrankung; Insulinresistenz; Diabetes Typ 2; Hypertonie; Schlafapnoe, Atemproblemen und/oder Dyslipidämie; Schlaganfall; Leber- und Gallenblasenerkrankung; Osteoarthritis; und/oder gynäkologischen Problemen.

2. Vitamin B2 zur Verwendung nach Anspruch 1, wobei das Vitamin B2 in der Form einer mütterlichen Nahrungsmittelzusammensetzung verabreicht wird.

3. Vitamin B2 zur Verwendung nach Anspruch 2, wobei die mütterliche Nahrungsmittelzusammensetzung ausgewählt ist aus der Gruppe bestehend aus einer pulverförmigen Nährstoffzusammensetzung zur Rekonstitution in Milch oder Wasser, einer Nährstoffformulierung, einem Produkt auf Getreidebasis, einem Getränk, einem Riegel, einem Nährstoffergänzungsmittel, einem Nutrazeutikum, einem Joghurt einem aus Milch gewonnenen Produkt, einer Nahrungsmittelbrause, einer Pille oder einer Tablette.

4. Vitamin B2 zur Verwendung nach einem der vorstehenden Ansprüche, wobei das Vitamin B2 in einer Menge verabreicht wird, die 0,14-14 mg Vitamin B2/Tag, zum Beispiel 1 -12,0 mg Vitamin B2/Tag, entspricht.

5. Vitamin zur Verwendung nach einem der vorstehenden Ansprüche, wobei das Vitamin B2 mindestens 4 aufeinanderfolgende Wochen vor Schwangerschaft, während Schwangerschaft und/oder während Stillzeit zu verabreichen ist.

6. Vitamin B2 zur Verwendung nach einem der vorstehenden Ansprüche, wobei das Vitamin B2 mindestens einmal täglich zu verabreichen ist.

7. Vitamin B2 zur Verwendung nach einem der vorstehenden Ansprüche, wobei das Vitamin B2 aus natürlichen Quellen, wie Milch, Fleisch, Geflügel, Eiern, Nüssen, Gemüse, Pilzen, Hefen, Nüssen und Erdnüssen; oder Extrakten und/oder Kombinationen davon bereitgestellt wird.

8. Vitamin B2 zur Verwendung nach einem der vorstehenden Ansprüche, wobei Übergewicht Fettleibigkeit, übermäßige Fettansammlung und damit verbundenen Stoffwechselstörungen bei den Nachkommen später im Leben vorgebeugt wird.

9. Vitamin B2 zur Verwendung nach Anspruch 8, wobei Übergewicht und/oder Fettleibigkeit bei den Nachkommen in dem Alter von mindestens 3 Jahren, zum Beispiel mindestens 4 Jahren oder mindestens 6 Jahren, vorgebeugt wird.

10. Vitamin B2 zur Verwendung nach einem der vorstehenden Ansprüche, wobei Übergewicht und/oder Fettleibigkeit durch Verringern von Fettmasse, insbesondere abdominaler und/oder viszeraler Fettmasse, vorgebeugt wird.

11. Vitamin B2 zur Verwendung nach einem der vorstehenden Ansprüche, wobei die Mutter eine multipare, übergewichtige und/oder fettleibige Mutter und/oder eine Mutter ist, die an Stoffwechselsyndrom leidet.

12. Mütterliche Nahrungsmittelzusammensetzung zur Verwendung bei der therapeutischen Vorbeugung von Übergewicht, Fettleibigkeit, übermäßiger Fettansammlung und Stoffwechselstörungen, die mit einem der Vorstehenden verbunden sind, bei den Nachkommen, wobei die mütterliche Nahrungsmittelzusammensetzung der Mutter vor Schwangerschaft, während Schwangerschaft und/oder während Stillzeit zu verabreichen ist, wobei damit verbundene Stoffwechselstörungen bei den Nachkommen ausgewählt sind aus der Gruppe bestehend aus: kardiovaskulären Erkrankungen, wie koronarer Herzerkrankung; Insulinresistenz; Diabetes Typ 2; Hypertonie; Schlafapnoe, Atemproblemen und/oder Dyslipidämie; Schlaganfall; Leber- und Gallenblasenerkrankung; Osteoarthritis; und/oder gynäkologischen Problemen, wobei die mütterliche Nahrungsmittelzusammensetzung eine pulverförmige Nährstoffformulierung ist, die eine Proteinquelle, eine Kohlenhydratquelle, eine Lipidquelle, Lecithin, gegebenenfalls aus Soja, Füllmittel und 0,1-9,8 mg Vitamin B2/100 g Trockengewicht umfasst, und wobei Vitamin B2 in der mütterlichen Nahrungsmittelzusammensetzung als Wirkstoff enthalten ist.

13. Mütterliche Nahrungsmittelzusammensetzung zur Verwendung nach Anspruch 12, ferner umfassend probiotische Bakterien, Folsäure, Calcium, Eisen, ARA, EPA und/oder DHA.

## Revendications

1. Vitamine B2 pour utilisation dans la prévention thérapeutique de surpoids, d'obésité, d'accumulation excessive de graisses et de troubles métaboliques associés à l'un ou l'autre de ce qui précède chez la progéniture, dans laquelle la vitamine B2 doit être administrée à la mère avant la grossesse, pendant la grossesse et/ou pendant l'allaitement dans laquelle les troubles métaboliques associés chez la progéniture sont choisis dans le groupe constitué de : maladies cardiovasculaires telles que maladie coronarienne ; insulinorésistance ; diabète de type 2 ; hypertension ; apnée du sommeil, problèmes respiratoires et/ou dyslipidémie ; accident vasculaire cérébral ; maladie du foie et de la vésicule ; arthrose ; et/ou problèmes gynécologiques.

2. Vitamine B2 pour utilisation selon la revendication 1, dans laquelle la vitamine B2 est administrée sous la forme d'une composition alimentaire maternelle.

3. Vitamine B2 pour utilisation selon la revendication 2, dans laquelle la composition alimentaire maternelle est choisie dans le groupe constitué d'une composition nutritionnelle en poudre à reconstituer dans du lait ou de l'eau, une préparation nutritionnelle, un produit à base de céréales, une boisson, une barre, un complément nutritionnel, un nutraceutique, un yaourt un produit dérivé de lait, un distributeur alimentaire, un comprimé ou une tablette.

4. Vitamine B2 pour utilisation selon l'une des revendications précédentes, dans laquelle la vitamine B2 est administrée en une quantité correspondant à 0,14 à 14 mg de Vitamine B2/jour, par exemple 1 à 12,0 mg de Vitamine B2/jour.

5. Vitamine pour utilisation selon l'une des revendications précédentes, dans laquelle la vitamine B2 doit être administrée pendant au moins 4 semaines consécutives avant la grossesse, pendant la grossesse et/ou pendant l'allaitement.

6. Vitamine B2 pour utilisation selon l'une des revendications précédentes, dans laquelle la vitamine B2 doit être administrée au moins une fois par jour.

7. Vitamine B2 pour utilisation selon l'une des revendications précédentes, dans laquelle la vitamine B2 est fournie à partir de sources naturelles telles que du lait, de la viande, de la volaille, des œufs, des fruits à coques, des légumes, des champignons, des levures, des fruits à coques et des arachides ; ou des extraits et/ou des combinaisons de ceux-ci.

8. Vitamine B2 pour utilisation selon l'une des revendications précédentes, dans laquelle le surpoids l'obésité, l'accumulation excessive de graisses et les troubles métaboliques associés sont empêchés chez la progéniture plus tard dans la vie.

9. Vitamine B2 pour utilisation selon la revendication 8, dans laquelle le surpoids et/ou l'obésité sont empêchés chez la progéniture à l'âge d'au moins 3 ans, par exemple au moins 4 ans ou au moins 6 ans.

10. Vitamine B2 pour utilisation selon l'une des revendications précédentes, dans laquelle le surpoids et/ou l'obésité sont empêchés en réduisant la masse graisseuse, en particulier la masse graisseuse abdominale et/ou viscérale.

11. Vitamine B2 pour utilisation selon l'une des revendications précédentes, dans laquelle la mère est une mère multipare, en surpoids et/ou obèse et/ou une mère souffrant d'un syndrome métabolique.

12. Composition alimentaire maternelle pour utilisation dans la prévention thérapeutique de surpoids, d'obésité, d'accumulation excessive de graisses et de troubles métaboliques associés à l'un ou l'autre de ce qui précède chez la progéniture, dans laquelle la composition alimentaire maternelle doit être administrée à la mère avant la grossesse, pendant la grossesse et/ou pendant l'allaitement dans laquelle les troubles métaboliques associés chez la progéniture sont choisis dans le groupe constitué de : maladies cardiovasculaires telles que maladie coronarienne ; insulinorésistance ; diabète de type 2 ; hypertension ; apnée du sommeil, problèmes respiratoires et/ou dyslipidémie ; accident vasculaire cérébral ; maladie du foie et de la vésicule ; arthrose ; et/ou problèmes gynécologiques, dans laquelle la composition alimentaire maternelle est une préparation nutritionnelle pulvérulente comprenant une source de protéines, une source de glucides, une source de lipides, de la lécithine éventuellement de soja, des agents gonflants et 0,1 à 9,8 mg de vitamine B2/100 g de poids sec, et dans laquelle la vitamine B2 est comprise dans la composition alimentaire maternelle en tant qu'ingrédient actif.

13. Composition alimentaire maternelle pour utilisation selon la revendication 12, comprenant en outre des bactéries probiotiques, de l'acide folique, du calcium, du fer, de l'ARA, de l'EPA et/ou du DHA.
